# EUROPEAN PATENT SPECIFICATION

(11) **EP 1 909 728 B1**
(45) Date of publication and mention of the grant of the patent: **28.09.2011**
(21) Application number: 06772921.0
(22) Date of filing: 09.06.2006
(51) Int. Cl.: A61F 13/56, A61F 13/62

(54) **DISPOSABLE ABSORBENT ARTICLE WITH FRONT FASTENING ASSEMBLY**
SAUGFÄHIGER EINWEG-ARTIKEL MIT VORDERER BEFESTIGUNGSANORDNUNG
ARTICLE ABSORBANT JETABLE DOTE D'UN ENSEMBLE D'ATTACHE AVANT

(30) Priority: 28.06.2005 US 169499
(43) Date of publication of application: 16.04.2008
(73) Proprietor: KIMBERLY-CLARK WORLDWIDE, INC., Neenah, WI 54956 (US)
(72) Inventor: VANGOMPEL, Paul, T., Hortonville, Wisconsin 54944 (US); ZEHNER, Georgia, L., Larsen, Wisconsin 54947 (US)
(74) Representative: Davies, Christopher Robert
(86) International application number: PCT/US2006/022814
(87) International publication number: WO 2007/001815

(56) References cited:
- US-A1- 2002 022 819
- US-A1- 2002 065 504
- US-A1- 2004 102 745
- US-B1- 6 174 303

## Description

### BACKGROUND OF THE INVENTION

The present invention relates to disposable absorbent articles which are adapted to contain body exudates. More particularly, the preset invention relates to disposable absorbent articles having a fastener located on a front waist region of the article.

Disposable garments, including disposable absorbent garments, have been known for decades. Disposable garments may include garments worn like underpants for children and adults, garments worn like training pants for toddlers and garments worn like diapers for infants. Disposable absorbent garments designed to absorb and contain bodily fluids may include adult/child incontinence garments, toddler training pants and infant diapers.

It is desirable for disposable garments to fit close to the body of the wearer for comfort and discretion. The disposable garment may include fasteners that assist with securing the position of the garment in use. For example, disposable garments may include adhesive or mechanical fasteners to assist with securing the garments around the waists of the users. During use, the fasteners undergo numerous stresses. As the user moves, by rolling, sitting, crawling and walking, the garments may deform, primarily in the front waist region as the angle between the user's legs and upper body increases and decreases. This movement and deformation may cause irritation to the user or may cause failure of the fastening system.

It is also desirable for disposable garments to be relatively easy to apply. Typically, disposable garments have outwardly extending tabs on the back portion of the garment. The tabs usually need to be unfolded before they are aligned with and fastened to a corresponding fastener located elsewhere on the garment. It may be difficult for some users to obtain optimum fit of a disposable garment if multiple steps are needed to ready the garment for fastening. One such step is the unfolding of tabs prior to fastening the garment.

Even though significant and numerous advancements have occurred in the materials and structural features available for the construction of disposable garments, there remains a need for garments which provide an optimum fit about the wearer and maintaining a pleasing appearance while being worn. Moreover, there remains a need for disposable absorbent articles which can be more easily secured about the waist of a wearer.

A prior art garment, having the features of the preamble of claim 1, is shown in US 2002/0022819.

### SUMMARY OF THE INVENTION

According to the present invention, there is provided a disposable garment as claimed in claim 1.

The present invention is directed to providing an absorbent article having a mechanical fastener disposed on the front waist region of the article, and that may connect with ear portions of the absorbent article. Varied peel and/or shear strengths may occur between the ear portions and the fastener assembly.

The present invention concerns a disposable garment having a front waist region, a rear waist region and a crotch region which extends between and connects the front waist region and the rear waist region. The garment includes a body-facing surface, a garment-facing surface, and a fastener assembly. The fastener assembly includes two side attachment zones spaced apart and disposed on the garment-facing surface of the front waist region, and a middle attachment zone disposed on the garment-facing surface between the two side attachment zones. The middle attachment zone contains at least one first fastener material, and the side attachment zones contain at least one second fastener material. The side attachment zones engage the body-facing surface at significantly different normalized peel strength than the middle attachments zone.

According to one embodiment, there is provided a disposable garment having a front waist region, a rear waist region and a crotch region which extends between and connects the front waist region and the rear waist region. The garment includes a body-facing surface, a garment-facing surface, and a fastener assembly. The fastener assembly includes two side attachment zones having a plurality of fastener components. The two side attachment zones are spaced apart and disposed on the garment facing surface of the front waist region. The fastener assembly further includes a middle attachment zone located between the two side attachment zones, the middle attachment zone containing at least one first fastener material and the side attachment zones containing at least one second fastener material. A pair of back ear portions extend from the rear waist region, and are configured so that they each engage at least a portion of the side attachment zones, and at least a portion of the middle attachment zone. The side attachment zones engage each of the pair of back ear portions at significantly different peel or shear strengths than the middle attachment zone.

Accordingly, this embodiment provides a disposable garment having side attachment zones that when attached to the back ear portions of the garment, form side seams that primarily maintain the fit of the garment while in use. The middle attachment zone is useful for adjusting the fit of the garment and for maintaining a more garment-like appearance. One possible feature of the garment is its potential to have the back ear portions made from the same body facing material as the remainder of the garment, so that they may be attached to the fastener assembly without having to unfold tabs or the like.

It is understood that both the foregoing general description and the following detailed description are exemplary and are intended to provide further explanation of the invention claimed. The accompanying drawings, which are incorporated in and constitute part of this specification, are included to illustrate and provide a further understanding of the article of the present invention. Together with the description, the drawings serve to explain the various aspects of the invention.

### BRIEF DESCRIPTION OF THE DRAWINGS

The present invention will be more fully understood and further advantages will become apparent when reference is made to the following detailed description of the invention and the accompanying drawings wherein like numerals represent like elements. The drawings are merely representative and are not intended to limit the scope of the appended claims.
Fig. 1 representatively shows a plan view of an example of a disposable absorbent article of Fig. 1 in an unfastened, stretched and laid flat condition with the surface of the article which contacts the wearer's skin facing the viewer and with portions of the article partially cut away to how the underlying features;
Fig. 2 representatively illustrates a perspective view of the disposable absorbent article (an infant diaper) of the disposable absorbent article of Fig. 1 in a semi-folded and unfastened condition;
Fig. 3 representatively illustrates a schematic plan view of the disposable absorbent article of Fig. 1 in an unfastened, stretched and laid flat condition with the surface of the article which contacts the wearer's clothing (exterior surface) facing the viewer;
Fig. 4 representatively illustrates a schematic front perspective view of the disposable absorbent article of Fig. 3 in a partially fastened condition;
Fig. 5 representatively illustrates a schematic plan view of a second embodiment of the disposable absorbent article in an unfastened stretched and laid flat condition with the surface of the article which contacts the wearer's clothing (exterior surface) facing the viewer;
Fig. 6 representatively illustrates a schematic front perspective view of the disposable absorbent article of Fig. 5 in a partially fastened condition;
Fig. 7 representatively illustrates a schematic plan view of a third embodiment of the disposable absorbent article in an unfastened stretched and laid flat condition with the surface of the article which contacts the wearer's clothing (exterior surface) facing the viewer; and
Fig. 8 representatively illustrates a schematic front perspective view of the disposable absorbent article of Fig. 7 in a partially fastened condition.

### DETAILED DESCRIPTION OF THE INVENTION

The present disclosure of the invention will be expressed in terms of its various components, elements, constructions, configurations, arrangements and other features that may also be individually or collectively be referenced by the term, "aspect(s)" of the invention, or other similar terms. It is contemplated that the various forms of the disclosed invention may incorporate one or more of its various features and aspects, and that such features and aspects may be employed in any desired, operative combination thereof.

It should also be noted that, when employed in the present disclosure, the terms "comprise" "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

Within the context of this specification, each term or phrase below includes the following meaning or meanings:
"Attach" and its derivatives refer to the joining, adhering, connecting, bonding, sewing together, or the like, of two elements. Two elements will be considered to be attached together when they are integral with one another or attached directly to one another or indirectly to one another, such as when each is directly attached to intermediate elements. "Attach" and its derivatives include permanent, releasable, or refastenable attachment. In addition, the attachment can be completed either during the manufacturing process or by the end user.
"Bond" and its derivatives refer to the joining, adhering, connecting, attaching, sewing together, or the like, of two elements. Two elements will be considered to be bonded together when they are bonded directly to one another or indirectly to one another, such as when each is directly bonded to intermediate elements. "Bond" and its derivatives include permanent, releasable, or refastenable bonding.
"Connect" and its derivatives refer to the joining, adhering, bonding, attaching, sewing together, or the like, of two elements. Two elements will be considered to be connected together when they are connected directly to one another or indirectly to one another, such as when each is directly connected to intermediate elements. "Connect" and its derivatives include permanent, releasable, or refastenable connection. In addition, the connecting can be completed either during the manufacturing process or by the end user.
The terms "disposed on," "disposed along," or "disposed toward" and variations thereof are intended to mean that one element can be integral with another element, or that one element can be a separate structure bonded to or placed with or placed near another element.
"Elastic," "elasticized," "elasticity," and "elastomeric" mean that property of a material or composite by virtue of which it tends to recover its original size and shape after removal of a force causing a deformation. Suitably, an elastic material or composite can be elongated by at least 25 percent (to 125 percent) of its relaxed length and will recover, upon release of the applied force, at least 40 percent of its elongation. Desirably an elastic material or composite be capable of being elongated by at least 100 percent (to 200 percent), more desirably by at least 150 percent (to 250 percent), of its relaxed length and recover, upon release of an applied force, at least 50 percent of its elongation.
"Hook and loop fasteners" refers to fastening means comprising a "hook" component and a complementary loop component. The term "hook" is used to designate a material having protruding or engaging elements. It should also be understood that the use of the term "hook" should be non-limiting in the sense that the engaging elements may comprise any shapes as are known in the art so long as they are adapted to engage a complementary fastener component or material characterized by loops or the like. "Layer" when used in the singular can have the dual meaning of a single element or a plurality of elements.
"Liquid impermeable," when used in describing a layer or multi-layer laminate means that liquid, such as urine, will not pass through the layer or laminate, under ordinary use conditions, In a direction generally perpendicular to the plane of the layer or laminate at the point of liquid contact.
"Liquid permeable" refers to any material that is not liquid impermeable.
"Nonwoven" and "nonwoven web" refer to materials and webs of material that are formed without the aid of a textile weaving or knitting process. For example, nonwoven materials, fabrics or webs have been formed from many processes such as, for example, meltblowing processes, spunbonding processes, air laying processes, and bonded carded web processes.
"Stretchable" means that a material can be stretched, without breaking, by at least 25 percent (to 125 percent of its initial (unstretched) length) in at least one direction, suitably by at least 100 percent (to 200 percent of its initial length), desirably by at least 150 percent (to at least 250 percent of its initial length) and may or may not recover properties upon release of an applied force. Elastic materials and extensible materials are each stretchable materials.
"Superabsorbent material" refers to a water-swellable, water-insoluble organic or inorganic material capable, under the most favorable conditions, of absorbing at least about ten times its weight and, more desirably, at least about thirty times its weight in an aqueous solution containing about 0.9 weight percent sodium chloride.

These terms may be defined with additional language in the remaining portions of the specification.

The present invention is directed to providing disposable garments having an improved fastening system. Additionally, the present invention is directed to a garment having a unique combination of features that provide previously unrecognized and unexpected benefits. This detailed description of the present invention will include a description of a representative disposable garment including the various components of such garments. The description of the representative disposable garment will also include a description of many features encompassed by the present invention.

### Representative Disposable Garment

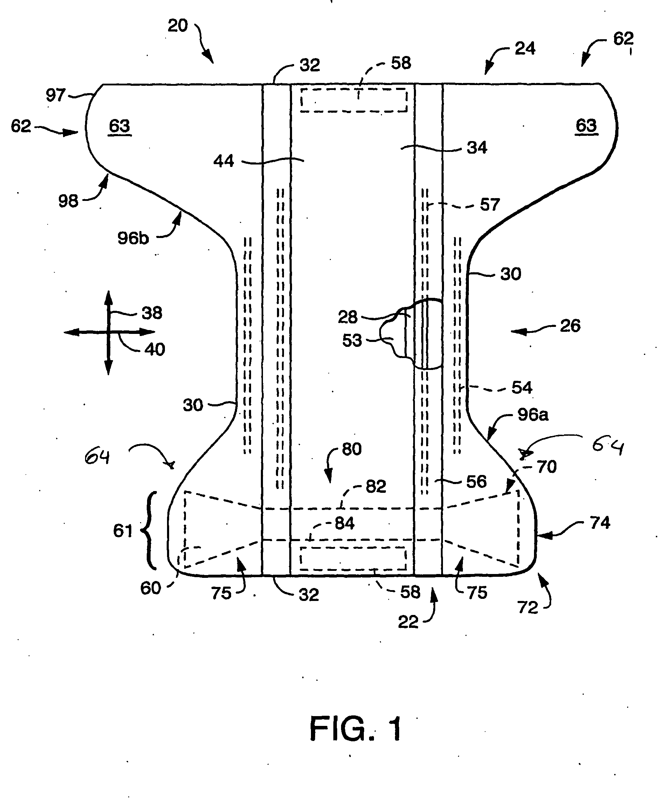

The absorbent articles of the present invention will be described in terms of a disposable diaper article and in terms of a diaper pant that is adapted to be worn by infants about the lower torso. The representative disposable garments illustrated in Figs. 1-8 each have a mechanical fastener assembly located on the front waist region of the garment. It is understood that the features of the present invention are equally adaptable for other types of absorbent articles such as adult incontinence garments, training pants, and disposable swim pants.

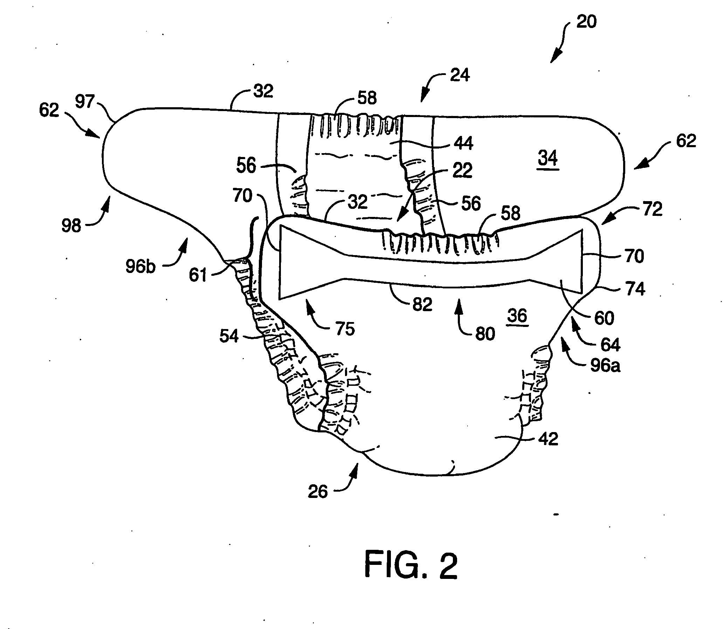

Fig. 1 representatively illustrates an example of a refastenable disposable diaper in an unfastened, stretched and laid flat configuration with the surface of the diaper adapted to contact the wearer's skin facing the viewer and with portions of the diaper partially cut away to show the underlying features. As illustrated in Fig. 1 and Fig. 2, the diaper pant or diaper 20 defines a front waist region 22, a back waist region 24, a crotch region 26 that extends between and connects the front and back waist regions 22 and 24, a longitudinal direction 38 and a lateral direction 40. The front waist region 22 includes the portion of the diaper 20 that, when worn, is positioned on the front of the wearer while the back waist region 24 includes the portion of the diaper 20 that, when worn, is positioned on the back of the wearer. The crotch region 26 of the diaper 20 includes the portion of the diaper 20 that, when worn, is positioned between the legs of the wearer and covers the lower torso of the wearer.

The diaper 20 defines a pair of laterally opposed side edges 30, a pair of longitudinally opposed waist edges 32, an interior surface 34 that is configured to contact the wearer, and an exterior surface 36 opposite the interior surface 34 that is configured to contact the wearer's clothing in use. The illustrated diaper 20 also includes a substantially liquid impermeable outer cover 42 and a liquid permeable bodyside liner 44 that can be connected to the outer cover 42 in a superposed relation. An absorbent core 28 is located between the outer cover 42 and the bodyside liner 44. The laterally opposed side edges 30 of the diaper 20 are generally defined by the side edges of the outer cover 42 that further define leg openings that may be curvilinear. The waist edges 32 of the diaper 20 are generally defined by the waist edges of the outer cover 42 and define a waist opening that is configured to encircle the waist of the wearer when worn. The absorbent core 28 is configured to contain and/or absorb body exudates discharged from the wearer. The diaper 20 may further include leg elastics 54, containment flaps 56 and waist elastics 58 as are known to those skilled in the art. It should be recognized that individual components of the diaper 20 may be optional depending upon the intended use of the diaper 20.

The diaper 20 may be of various suitable shapes. For example, in the unfastened configurations as illustrated in Fig. 1, the diaper 20 may generally have an overall rectangular shape, T-shape, I shape or an approximately hourglass shape. In the shown embodiments, the diaper 20 has a generally I-shape in an unfastened configuration.

The various components of the diaper 20 are integrally assembled together employing various types of suitable attachment means, such as adhesive, sonic and thermal bonds or combinations thereof. In the shown embodiments, for example, the outer cover 42 and bodyside liner 44 are assembled to each other and to the absorbent core 28 with adhesive, such as a hot melt, pressure-sensitive adhesive. The adhesive may be applied as a uniform continuous layer of adhesive, a patterned layer of adhesive, a sprayed pattern of adhesive, or an array of separate lines, swirls or dots of adhesive. Alternatively, the absorbent core 28 may be connected to the outer cover 42 using conventional fasteners such as buttons, hook and loop type fasteners, adhesive tape fasteners, and the like. The other components of the diaper 20 may be suitably connected together using similar means. Similarly, other diaper components, such as the elastic members 54 and 58 and the mechanical fasteners (described herein), may be assembled into the diaper 20 article by employing the above-identified attachment mechanisms. Desirably, the majority of the diaper components are assembled together using ultrasonic bonding techniques for reduced manufacturing cost.

The outer cover 42 of the diaper 20, as representatively illustrated in Fig. 2, may suitably be composed of a material which is either liquid permeable or liquid impermeable. It is generally preferred that the outer cover 42 be formed from a material that is substantially impermeable to liquids. A typical outer cover 42 can be manufactured from a thin plastic film or other flexible liquid-impermeable material. For example, the outer cover 42 may be formed from a polyethylene film having a thickness of from about 0.013 millimeter (0.5 mil) to about 0.051 millimeter (2.0 mils). The materials of the outer cover 42 can be thermally or adhesively laminated together. Suitable laminate adhesives, which can be applied continuously or intermittently as beads, a spray, parallel swirls, or the like, can be obtained from Bostik-Findley, Inc., of Wauwatosa, Wisconsin, U.S.A., or from National Starch and Chemical Company, Bridgewater, New Jersey, U.S.A. If It is desired to present the outer cover 42 with a more clothlike feeling, the outer cover 42 may be formed from a polyolefin film having a nonwoven web laminated to the exterior surface thereof, such as a spunbond web of polyolefin fibers. For example, a stretch-thinned polypropylene film having a thickness of about 0.015 millimeter (0.6 mil) may be thermally laminated thereto a spunbond web of polypropylene fibers. The polypropylene fibers may have a fiber diameter of about 15 to 20 microns, which nonwoven web has a basis weight of about 17 grams per square meter (0.5 ounce per square yard). The outer cover 42 may include bicomponent fibers such as polyethylene/polypropylene bicomponent fibers. Methods of forming such clothlike outer covers are known to those skilled in the art, The outer cover 42 may also be an extensible outer cover such as the outer covers described in U.S. Patent No. 6,562,245 issued on April 22, 2003 to Roessler et al. The outer cover 42 may also be a biaxially stretchable outer cover such as the outer covers described in U.S. Patent No. 6,702,800 issued on March 9, 2004 by Vukos et al.

The outer cover 42 may be formed of a woven or nonwoven fibrous web layer which has been totally or partially constructed or treated to impart a desired level of liquid impermeability to selected regions that are adjacent or proximate the absorbent core 28. Still further, the outer cover 42 may optionally be composed of a micro-porous "breathable" material which permits vapors to escape from the absorbent core 28 while still preventing liquid exudates from passing through the outer cover 42. For example, the outer cover 42 may include a vapor permeable non-woven facing layer laminated to a micro-porous film. Suitable "breathable" outer cover materials are described in U.S. Patent No. 5,695,868 issued December 9, 1997 to McCormack et al. and U.S. Patent No. 5,843,056 issued December 1, 1998 to Good et al. Still further, the outer cover 42 may also be an elastomeric material such as a stretch-thermal laminate (STL), neck-bonded laminate (NBL), or stretch-bonded laminate (SBL) material. Methods of making such materials are well known to those skilled In the art and are described in U.S. Patent No. 4,663,220 issued May 5, 1987 to Wisneski et al., U.S. Patent No. 5,226,992 issued July 13,1993 to Morman, and European Patent Application No. EP 0 217 032 published on April 8, 1987 in the names of Taylor et al. The outer cover 42 can also be embossed or otherwise provided with a matte finish to provide a more aesthetically pleasing appearance.

In order to reduce the perception that the outer cover 42 feels damp or clammy, the diapers/diaper pants 20 of the invention may include a spacer or ventilation layer (not shown in Figures) between the garment-facing surface of the absorbent core 28 and the outer cover 42. The ventilation layer may include one or more nonwoven materials, for example a spunbond-meltblown-spunbond nonwoven material.

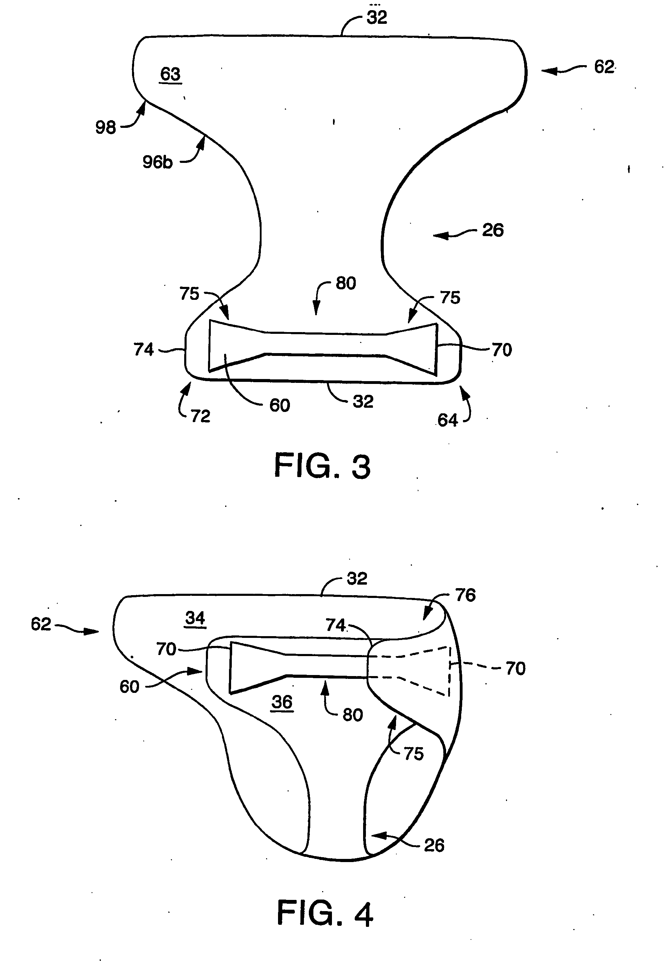

The representative absorbent articles of the invention include a bodyside liner 44 in superimposed relation to the outer cover 42. The bodyside liner 44, as representatively illustrated in Fig. 2 and Fig. 4, suitably presents a bodyfacing surface that is compliant, soft feeling, and nonirritating to the wearer's skin. Further, the bodyside liner 44 may be less hydrophilic than the absorbent core 28, to present a relatively dry surface to the wearer, and may be sufficiently porous to be liquid permeable, permitting liquid to readily penetrate through its thickness. A suitable bodyside liner 44 may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. The bodyside liner 44 is suitably employed to help isolate the wearer's skin from liquids held in the absorbent 28. The bodyside liner 44 can also be made from extensible materials as are described in U.S. Patent No. 6,552,245 issued on April 22, 2003 to Roessler et al, The bodyside liner 44 can also be made from biaxially stretchable materials as are described in U.S. Patent No. 6,702,800 issued on March 9, 2004 by Vukos et al.

Various woven and nonwoven fabrics can be used for the bodyside liner 44. For example, the bodyside liner may be composed of a meltblown or spunbond web of polyolefin fibers. The bodyside liner 44 may also be a bonded-carded web composed of natural and/or synthetic fibers. The bodyside liner 44 may be composed of a substantially hydrophobic material, and the hydrophobic material may optionally be treated with a surfactant or otherwise processed to impart a desired level of wettability and hydrophilicity. In a particular embodiment of the present invention, the bodyside liner 44 is made from a nonwoven, spunbond, polypropylene fabric composed of fibers having a fiber diameter of about 21 to 23 microns formed into a web having a basis weight of about 20 grams per square meter and a density of about 0.13 grams per cubic centimeter. The fabric may be surface treated with about 0.3 weight percent of a surfactant, such as a surfactant commercially available from Hodgson Textile Chemicals, Inc, under the trade designation AHCOVEL Base N-62. The surfactant may be applied by any conventional means, such as spraying, printing, brush coating or similar techniques. The surfactant may be applied to the entire bodyside liner 44 or may be selectively applied to particular sections of the bodyside liner 44, such as the medial section along the longitudinal centerline of the diaper, to provide greater wettability of such sections. The bodyside liner 44 may further include a lotion or treatment applied thereto that is configured to be transferred to the wearer's skin. Suitable compositions for application to the bodyside liner 44 are described in U.S. Patent No. 6,149,934 that issued to Krzysik et al. on November 21, 2000.

The representative absorbent articles of the invention can include an absorbent core 28 disposed between the outer cover 42 and the bodyside liner 44. The absorbent core 28 of the diaper 20, as representatively illustrated in Fig. 1 and Fig. 3, may suitably include a matrix of hydrophilic fibers, such as a web of cellulosic fluff, mixed with particles of a high-absorbency material commonly known as superabsorbent material. In a particular aspect, the absorbent core 28 includes a matrix of cellulosic fluff, such as wood pulp fluff, and superabsorbent hydrogel-forming particles. The wood pulp fluff may be exchanged with synthetic, polymeric, meltblown fibers or with a combination of meltblown fibers and natural fibers. The superabsorbent particles may be substantially homogeneously mixed with the hydrophilic fibers or may be nonuniformly mixed. Alternatively, the absorbent core 28 may include a laminate of fibrous webs and superabsorbent material or other suitable matrix for maintaining a superabsorbent material in a localized area.

The absorbent core 28 may have any of a number of shapes. For example, the absorbent core 28 may be rectangular, I-shaped, or T-shaped. It is generally preferred that the absorbent core 28 is narrower in the intermediate section than in the front or rear waist sections of the diaper 20. The absorbent core 28 may be provided by a single layer or, in the alternative, may be provided by multiple layers, all of which need not extend the entire length and width of the absorbent core 28. In a particular aspect of the invention, the absorbent core 28 can be generally T-shaped with the laterally extending cross-bar of the "T' general ly corresponding to the front waist region 22 of the absorbent article for improved performance, especially for male infants.

The size and the absorbent capacity of absorbent core 28 should be compatible with the size of the intended wearer and the liquid loading imparted by the intended use of the absorbent article. Further, the size and the absorbent capacity of the absorbent core 28 can be varied to accommodate wearers ranging from infants through adults. In addition, it has been found-that with the present invention, the densities and/or basis weights of the absorbent core 28 can be varied.

The high-absorbency material may be selected from natural, synthetic, and modified natural polymers and materials. The high-absorbency materials may be inorganic materials, such as silica gels, or organic compounds, such as crosslinked polymers. The term "crosslinked" refers to methods for effectively rendering normally water-soluble materials substantially water insoluble but swellable. Such methods include, for example, physical entanglement, crystalline domains, covalent bonds, ionic complexes and associations, hydrophilic associations such as hydrogen bonding, and hydrophobic associations or Van der Waals forces.

Examples of synthetic, polymeric, high-absorbency materials include the alkali metal and ammonium salts of poly(acrylic acid) and poly(methacrylic acid), poly(acrylamides), poly(vinyl ethers), maleic anhydride copolymers with vinyl ethers and alpha-olefins, poly(vinyl pyrrolidone), poly(vinyl morpholinone), poly(vinyl alcohol), and mixtures and copolymers thereof. Further polymers suitable for use in the absorbent core 28 include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum, and the like. Mixtures of natural and wholly or partially synthetic absorbent polymers can also be useful in the present invention.

The high absorbency material may be in any of a wide variety of geometric forms. As a general rule, it is preferred that the high absorbency material be in the form of discrete particles. However, the high absorbency material may also be in the form of fibers, flakes, rods, spheres, needles, or the like. In general, the high absorbency material is present in the absorbent core 28 in an amount of from about 5 to about 90 percent by weight, desirably in an amount of at least about 30 percent by weight, and even more desirably in an amount of at least about 50 percent by weight based on a total weight of the absorbent core 28. For example, in a particular aspect, the absorbent core 28 may include a laminate which includes at least about 50 percent by weight and desirably at least about 70 percent by weight of high-absorbency material overwrapped by a fibrous web or other suitable material for maintaining the high-absorbency material in a localized area.

Optionally, a substantially hydrophilic tissue or nonwoven wrapsheet (not illustrated) may be employed to help maintain the integrity of the structure of the absorbent core 28. The wrapsheet is typically placed about the absorbent core 28 over at least the two major facing surfaces thereof. The wrapsheet may be composed of an absorbent cellulosic material, such as creped wadding or a high wet-strength tissue. In one aspect of the invention, the wrapsheet may be configured to provide a wicking layer that helps to rapidly distribute liquid over the mass of absorbent fibers constituting the absorbent core 28.

Due to the thinness of absorbent core 28 and the high absorbency material within the absorbent core 28, the liquid uptake rates of the absorbent core 28, by itself, may be too low, or may not be adequately sustained over multiple insults of liquid into the absorbent core 28. To improve the overall liquid uptake and air exchange, the diaper 20 of the different aspects of the present invention may further include a porous, liquid-permeable layer of surge management material 53, as representatively illustrated in Fig. 1. The surge management layer 53 is typically less hydrophilic than the absorbent core 28, and has an operable level of density and basis weight to quickly collect and temporarily hold liquid surges, to transport the liquid from its initial entrance point and to substantially completely release the liquid to other parts of the absorbent core 28. This configuration can help prevent the liquid from pooling and collecting on the portion of the diaper 20 positioned against the wearer's skin, thereby reducing the feeling of wetness by the wearer. The structure of the surge management layer 53 also generally enhances the air exchange within the diaper 20.

Various woven and nonwoven fabrics can be used to construct the surge management layer 53. For example, the surge management layer 53 may be a layer composed of a meltblown or spunbond web of synthetic fibers, such as polyolefin fibers. The surge management layer 53 may also be a bonded-carded-web or an airlaid web composed of natural and synthetic fibers. The bonded-carded-web may, for example, be a thermally bonded web that is bonded using low melt binder fibers, powder or adhesive. The webs can optionally include a mixture of different fibers. The surge management layer 53 may be composed of a substantially hydrophobic material, and the hydrophobic material may optionally be treated with a surfactant or otherwise processed to impart a desired level of wettability and hydrophilicity. In a particular aspect, the surge management layer 53 includes a hydrophobic, nonwoven material having a basis weight of from about 30 to about 120 grams per square meter.

The absorbent articles of the invention can include additional components. For example, as representatively illustrated in Figs. 1-4, the disposable diaper 20 may include a pair of containment flaps 56 that are configured to provide a barrier to the lateral flow of body exudates. The containment flaps 56 may be located along the laterally opposed side edges 30 of the diaper adjacent the side edges of the absorbent core 28. Each containment flap 56 typically defines an unattached edge that is configured to maintain an upright, perpendicular configuration in at least the crotch region 26 of the diaper 20 to form a seal against the wearer's body. The containment flaps 56 may extend longitudinally along the entire length of the absorbent core 28 or may only extend partially along the length of the absorbent core 28. When the containment flaps 56 are shorter in length than the absorbent core 28, the containment flaps 56 can be selectively positioned anywhere along the side edges 30 of diaper 20 in the crotch region 26. In a particular aspect of the invention, the containment flaps 56 extend along the entire length of the absorbent core 28 to better contain the body exudates. Such containment flaps 56 are generally well known to those skilled in the art.

The diaper 20 of the different configurations of the present invention may further include elastics at the waist edges 32 and side edges 30 of the diaper 20 to further prevent leakage of body exudates and support the absorbent core 28. For example, as representatively illustrated in Figs. 1-4, the diaper 20 of the present invention may include a pair of leg elastic members 54 that are connected to the literally opposed side edges 30 of the diaper 20 In the crotch region 26. The diaper 20 may also include a pair of waist elastic members 58 that is connected to the longitudinally opposed waist edges 32 of the diaper 20. The leg elastics 54 and waist elastics 58 are generally adapted to fit about the legs and waist of a wearer in use to maintain a positive, contacting relationship with the wearer to effectively reduce or eliminate the leakage of body exudates from the diaper 20.

Materials suitable for use as the leg elastics 54 and waist elastics 58 are.. well known to those skilled in the art. Exemplary of such materials are sheets or strands or ribbons of a polymeric, elastomeric material that may be adhered to the outer cover 42 in a stretched position, or that may be attached to the outer cover 42 while the outer cover is pleated, such that elastic constrictive forces are imparted to the outer cover 42. The leg elastics 54 may also include such materials as polyurethane, synthetic and natural rubber. The waist elastics 58 may be formed by elastic strands attached to the outer cover 42 or they may be formed by attaching separate pieces of stretchable materials to the waist regions of the article. For example, the waist elastics 58 may include a piece of stretch-bonded laminate material attached to the interior surface 34 of the article to form a waistband. Elasticity may be added or incorporated into the waist opening of absorbent articles utilizing a variety of known approaches.

Generally, the diaper pant chassis may be similar to the chassis described in U.S. Patent Application Serial Nos. 10/750,253 filed December 31, 2003, and 101879,323 filed June 29, 2004 published as EP 1 708 661.

The absorbent articles of the present invention include a refastenable mechanical fastener assemble 60 as described below, that can operate in conjunction with one or more components which may extend laterally outward from the longitudinal sides of the article.

Each waist region 22, 24 may have a pair of ear portions extending therefrom. Thus, each waist region 22, 24 defines a center portion between lateral ear portions, namely, back ear portions 62 and front ear portions 64, respectively. The front ear portions 64 and the back ear portions 62 can be used to improve the fit of the absorbent article. For instance, the front ear portions 64 may provide additional coverage around the waist of the wearer and they may assist caregivers with positioning the front waist region 22 on the wearer of the article. The back ear portions 62 may also provide coverage around the waist of the wearer. More specifically, the back ear portions 62 may provide the bridging material between the back waist region 24 of the article and the front waist region 22 such that the back ear portions 62 form part of the article's waist opening and an upper edge of the article's leg openings.

Referring to FIG. 1, each back ear portion 62 is connected to waist region 24 at a proximal region 96b. The opposite distal region 98 defined by an outer edge 97. Each back ear portion 62 further has an inner surface 63 that can make contact with the wearer's body when the diaper is worn, a body-facing surface. Similar to the back ear portion 62, the front ear portions 64 have a proximal region 96a located near a waist region 22. The outermost part of the front ear portions 64 are distally located end margins 72 defined by outer edges 74.

The back ear portions 62 may include or be comprised of material that is stretchable and extensible material, or material that is non-stretchable or non-extensible. Further, back ear portions 62 may be integral with the remaining portion of the diaper 20 as shown, or be a separate components that are attached to the diaper 20 (not shown). If back ear portions 62 and front ear portions 64 are formed integrally, they may be defined by the outer cover 42 and/or the bodyside liner 44 materials.

The back ear portions 62 may or may not have a separate mechanical fastener component attached thereto. Such components may be tab members (not shown) that extend outwardly in a substantially lateral direction from each back ear portion 62. Like the ears, the tabs may be integral, attached, stretchable and/or extensible, or neither stretchable nor extensible.

Back ear portions 62 may include a fastener material that is attached to the inner ear surface 63, which does not extend outwardly from the ear portion 62 in a plane that extends in the longitudinal direction 38 and lateral direction 40. Suitably, the back ear portions 62 do not include separate tabs or mechanical fastener components, and instead have a body facing surface 63 that is capable of directly engaging the mechanical fastener assembly 60. The advantage this provides is that the wearer does not have to purposefully match up a fastener on the back ear portion 62 with the mechanical fastener assembly 60. For instance, caretakers of children do not have to search for a tab or back ear fastening component when changing a diaper, and an adult with limited mobility or sight does not have to feel or look for a tab or back ear fastening component when applying an incontinence garment.

Desirably, back ear portions 62 may be comprised of a material that can engage a mechanical fastener assembly 60 located on the front waist region 22 (see Fig. 1). An exemplary material from which the back ear portions 62 may be constructed is a necked bonded laminate with two nonwoven (e.g. spunbond) facings and an elastomeric film (e.g. KRATON film) laminated therebetween. Other suitable stretchable materials are known in the art. When the back ear portions 62 include a stretchable material, the article may be worn by a greater range of users as a result of the increased fit range. Depending on the design of the article, it may also be desirable for the front ear portions 64 to include a stretchable material.

The shape and size of the front and back ear portions 62, 64 may vary from what is shown by way of example in the Figures. Further, when the product form of the absorbent article is a training pant or a swim pant, the back ear portions 62 and the front ear portions 64 are understood to include the side panels that are attached to the longitudinal sides 30 of the article and also are attached to each other to form side seams of the article. Typically, the side panels of training pants and swim pants are made from stretchable materials. The side panels' ability to stretch allows these products to be pulled on the wearer like underpants.

The mechanical fastener assembly 60 releasably engages the back ear portions 62 of the diaper 20. Generally, fastener assembly 60 is an elongated member defined by a pair of outer regions 75 that are separated by a middle portion 80. The outermost lateral edges of each outer region 75 are defined by distal edges 70. In the longitudinal direction 38, the longitudinal length of the distal edge 70 may be greater than the longitudinal length of the middle portion 80. Suitably, the length of the middle portion 80 may gradually increase in length from an inward edge 82 until it reaches the length of distal edge 70. Likewise, the length of the middle portion 80 may gradually increase in length from an outward edge 83 until it reaches the length of distal edge 70. As described herein, the fastener assembly 60 may have various configurations, and have single or multiple components.

One embodiment of the fastener assembly 60 is illustrated in Figs. 1-4. In this particular embodiment, the fastener assembly 60 has a unitary and symmetric construction. Fastener assembly 60 is generally a bow-tie shaped elongated member that is positioned laterally between front outer edges 74 of ear portions 64. Fastener assembly 60 has two distal ends 70 that may extend out to front end margins 72 of the garment, or extend completely to the front outer edges 74 of the garment.

Desirably, in the longitudinal direction 38, the length of each distal edge 70 is such that a side seam 76 is formed when the diaper 20 is in a fastened position. For any of the embodiments described herein, side seam 76 is defined by the bond between the front waist region 22 and the back waist region 24. Side seam 76 is the primary fastening or bond that fits the diaper 20 to the wearer's body. Side seam 76 may coincide with the wearer's general hip region, and may either be positioned like a seam found in a pair of traditional cloth underpants, or be located more forward or backward than a seam found in a pair of traditional cloth underpants.

Still referring to Figs. 1-4, the fastener middle portion 80 has a length measured at the longitudinal centerline or in the longitudinal direction 38, and a width measured in lateral direction 40. The width of middle portion 80 may be about 70 percent of the distance between each distal end 70. The middle portion 80 may have a length that is less than the length of each distal edge, the outermost edge. The purpose of having a shorter middle portion 80 as compared to the distal edge 70 is to prevent the diaper 20 from drooping or being pushed down by the wearer's belly. An exemplary shape of the fastener assembly 60 is such that the middle portion 80 is rectangular, however, other shapes are possible, e.g. hourglass, oval, etc. For example, near the outer edge,of containment flaps 56, the length of the middle portion 80 may increase linearly from the inward edge 82 and outward edge 83 until it reaches the length of distal edge 70. However, it is not important that the fastener assembly 60 have the exact shape as shown in Figs 1-4. Rather, it is desirable that the middle portion 80 have a length of about 1.2 cm to about 7.6 cm, (0,5 inches to about 3 inches) and the distal ends 70 have a length of about about 3.8- cm to about 20.3 cm, (1.5 inches to about 8 inches). More suitably, it is desirable that the middle portion 80 have a length bf about 2.5 cm to about 5.1 cm (1 inches to about 2 inches) and the distal ends 70 have a length of about about 5.1 cm to about 15.2. cm, (2 inches to about 6 inches). Even suitably, it is desirable that the middle portion 80 have a length of about 3.2 cm to about 3.8 cm, (1.25 inches to about 1.5 inches) and the distal ends 70 have a length of about(6.4 cm to about 12.7 cm, (2.5 inches to about 5 inches). In the alternative, the perimeter defining the overall fastener assembly 60 may be an I-shape, hourglass shape, or any other shape that is greater in length on the distal edges 70 than in the middle portion 80. Further, the fastener assembly 60 may be symmetric about the longitudinal axis of diaper 20 without being symmetric in any other direction.

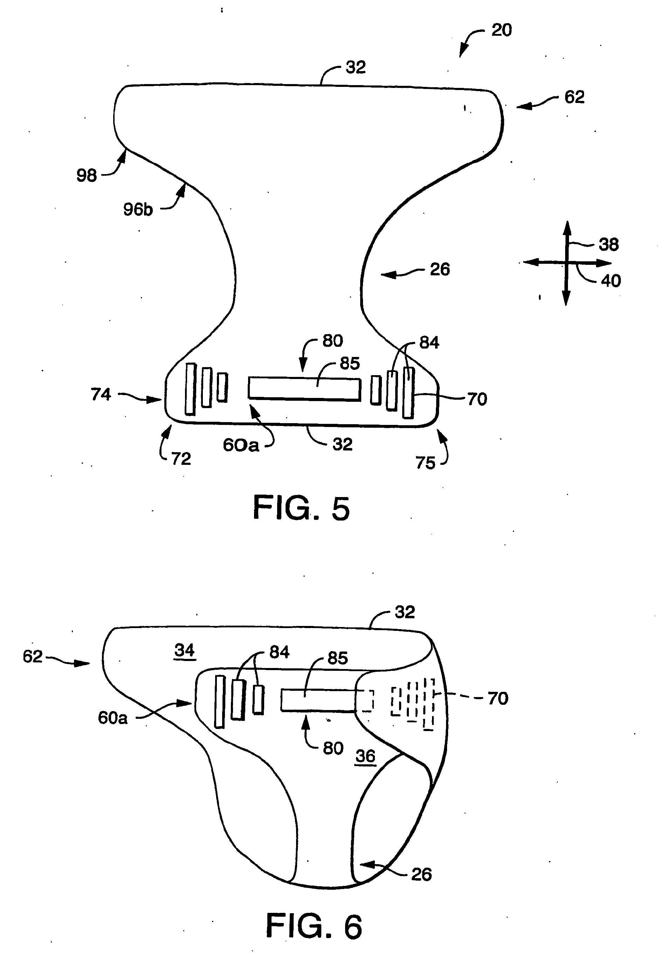

Alternatively, as shown in Figs. 5-8, fastener assembly 60 may have multiple fastening elements. Referring now to Figs. 5 and 6, shown is a second embodiment of the fastener assembly 60, referred to as fastener assembly 60a. Fastener assembly 60a generally differs from fastener assembly 60 in that it is discontinuous. As shown by way of example, fastener assembly 60a is comprised of separate fastener components 84 that may vary in size and a central component 85. Suitably, within a particular waist region, components 84 that are located inboard are shorter than the components located outboard. For example, referring to Fig. 6, if there are three components 84 corresponding to each front ear portion 64, the longest component with respect to the longitudinal direction 38 will be component 84a. Component 84a will have an outermost edge that defines distal edge 70. The shortest component may be component 84c, located adjacent central component 85. The mid-length component may be component 84b, disposed in a lateral space between components 84a and 84c. Most suitably, component 84a defines the length of side seam 76 when the diaper 20 is in a fastened condition. Components 84 a-c may be oriented to that the longitudinal axis of each such component is substantially aligned with longitudinal direction 38. Central component 85 may be a single elongated piece of material, disposed on diaper 20 so that its longest axis is in the lateral direction 40. Suitably, central component 85 is spaced laterally from each component 84c.

Though shown as being comprised of seven separate components, fastener assembly 60a may be comprised of more or less number of components. In addition, the shape of each separate component 84 and 85 need not be rectangular as shown, but could consist of a series of dots, ovals, lines (either straight or curved), decorative shapes, and the like.

Overall, the general shape of fastener assembly 60a may be the same as the fastener assembly 60 shown in Fig. 1. Alternatively, the fastener assembly 60a may have an overall I-shape, hourglass shape, or any other continuous shape that is greater in length on the distal edges 70 than in the middle portion 80. The average longitudinal length of components 84a-c may be greater than the longitudinal length of middle portion 80, as measured at the longitudinal axis of the garment, by as little as about 40%, and as much as about 60%. More suitably, little as about 30% and as much as about 70%; and even more suitably, as little as about 20%, and so much as about 130%. Further, the fastener assembly 60a may be symmetric about the longitudinal axis of diaper 20 without being symmetric in any other direction.

The width of the various components as measured in lateral direction 40 may vary depending on the fastener material properties and the size range the diaper is intended to cover. In particular, the width of center component 85 may be such that the back ear portion 62 can be fastened thereto until the wearer is in need of a size change. Thus, the back ear portions 62 may overlap, or may cover about 50 percent of the width of the center component 85 to as little as about 15 percent of the center component 185. Further, one ear portion may cover a significantly larger area of center component than the other ear portion, for example a 90/10 ratio of coverage could be possible. The width of components 84 may vary from component to component or be equivalent. Suitably, the width of components 84 is about 10 mm to about 30 mm; more suitably about 5mm to about 20mm, and even more suitably about 3 mm to about 10 mm.

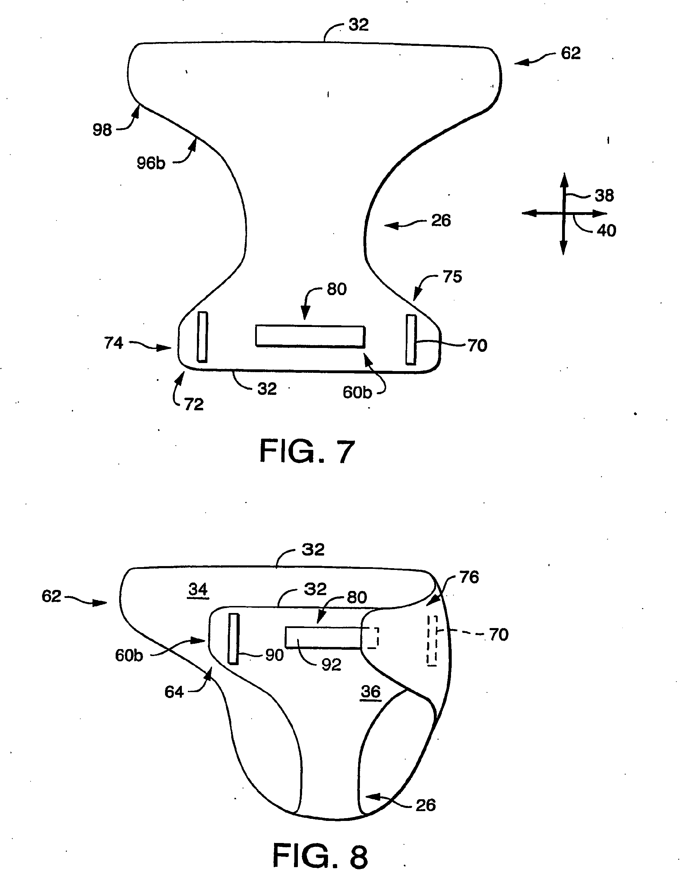

Referring now to Figs. 7 and 8, shown is a third embodiment of the fastener assembly 60, referred to as fastener assembly 60b. Fastener assembly 60 generally differs from fastener assembly 60 in that it is discontinuous, and differs from fastener assembly 60a in that is comprised of three separate components, namely, outer components 90 and central component 92.

Outer components 90 define side seam 76 when the diaper 20 is in a fastened condition. Outer components may be substantially aligned with the longitudinal direction 38, whereas central component may be substantially aligned with the lateral direction 40.

As with the previous embodiment, the shape of each separate component 90, 92 need not be rectangular as shown, but could consist of a series of dots, ovals, triangles, lines (either straight or curved), decorative shapes, and the like. Because there are only three components in this particular embodiment, fastener assembly 60b will generally have an overall I-shape wherein the distal edges 70 are greater in length in the longitudinal direction 38 than the middle portion 80. Suitably, it is desirable that the middle portion 80 have a length of about 1.2 cm to about 7.6 cm, (0.5 inches to about 3 inches) and the distal ends 70 have a length of about 3.8- cm to about 20.3 cm, (1.5 inches to about 8 inches). More suitably, it is desirable that the middle portion 80 have a length of about 2.5 cm to about 5.1 cm (1 inches to about 2 inches) and the distal ends 70 have a length of about 5.1 cm to about 15.2 cm, (2 inches to about 6 inches). Even suitably, it is desirable that the middle portion 80 have a length of about 3.2 cm to about 3.8 cm, (1.25 inches to about 1.5 inches) and the distal ends 70 have a length of about(6.4 cm to about 12.7 cm, (2.5 inches to about 5 inches

The width of the various components may vary depending on the fastener material properties and the size range the diaper is intended to cover. In particular, the width of center component 92 may be such that the back ear portion 62 can be fastened thereto until the wearer is in need of a size change. Thus, the back ear portion 62 may cover about 50 percent of the width of the center component 92 or possibly overlap as described above for the previous embodiment. Or, back ear portion 62 may each cover as little as about 15 percent of the center component 92. Suitably, the width of outer component may be about 5mm to about 25mm; more suitably about 10mm to about 20mm, and even more suitably about 12mm to about 15mm.

Regardless of the embodiment of the fastener assembly as shown in Figs. 1-8, it is desirable that refastenable connection between the back ear portion 62 and the fastener assembly (60 - 60b) vary in peel shear strength depending on which portion of the fastener assembly is contacting the back ear portion 62. The proximal region 96b of back ear portion 62 attaches to the outer region 75 of fastener assembly 60 - 60b to form the refastenable side seam 76 that is characterized by a primary peel and shear strength. The distal region 98 of back ear portion 62 forms a secondary bond with the middle or central portion of the fastener assembly that is used to adjust the fit of the diaper/pant to the wearer's body. Thus, the distal region 98 of back ear portion 62 has a refastenable connection with the middle or central component (80, 185 or 92) of the fastener assembly 60 - 60b characterized by a secondary peel and shear strength.

Peel and shear strengths between fastener assembly component a nd back ear portion may be measured and the resulting strengths "normalized" to account for fastener surface area, aspect ratio, and/or length. Using test techniques as described herein for hook-and-loop fasteners (or comparable techniques for non hook-and-loop fasteners), the normalized primary peel strength may be greater than the normalized secondary peel strength by as much as about 10 to about 200 percent, more suitably by as much as about 10 to about 75 percent, and even more suitably by as much as about 15 to about 50 percent. The primary shear strength may be greater than the secondary shear strength by as much as about 10 to about 200 percent, and more suitably by as much as about 20 to about 150 percent, and even more suitably by as much as about 30 to about 100 percent.

The peel strength of a fastener is directly proportional to the length of the fastener, wherein the fastener length is measured in a direction perpendicular to the peel force. For example, if the length of a primary fastener is 7.6 cm (3 inches) and the length of a secondary fastener made from the same material 2.5 cm is (1.0 inch), the force required to peel the primary fastener from a material would be about 3 times the force required to peel the secondary fastener from the same material.

The shear strength of a fastener is related the surface area and aspect ratio of the contacting portion of the fastening assembly and back ear portion. When the surface area of the primary fastener is greater than the surface area of the secondary fastener, the primary fastener will exhibit greater shear strength than the secondary fastener when the aspect ratios and materials are equivalent. For example, a square fastening component with an area of 5.08 cm square (2 Inch square) may have about twice the shear strength of a square fastening component with an area of 2.54 cm square (1.0 inch square). If the area is held constant, and the aspect ratio is such that the same fastening material is rectangular, for example 2:1, it may exhibit a lower or higher shear strength depending on how the rectangular fastener is oriented with respect to an applied shear force.

### Peel Strength Test

This procedure is a tensile bench test to measure the peel force required to separate a mechanical fastening system that joins two materials. The peel force of separation is measured by determining load values as the two materials are pulled apart perpendicular to their plane of contact. The direction of removal (peel), in this application, is that direction in which the fastener material would generally be removed from a substrate when the product is in use. Peel strength is normalized by dividing by the contact area resulting in a force per area.

### Equipment

1. Tensile tester capable of obtaining a peak load and equipped with an appropriate load cell. A suitable tensile testing system is a Sintech Tensile Tester, commercially available from MTS Sintech, Research Triangle Park, North Carolina, under the trade designation instron Model 4201 Tensile Tester with Sintech QAD (Quality Assurance Department) Software.
2. Software commercially obtained from MTS Sintech under the trade designation Sintech Testworks®.
3. Pnuematic-action grips commercially available from Instron Corporation, Canton, Massachusetts, under the trade designation "Instron Model 2712-004."
4. 1 by 4 inch grip faces, serrated, commercially available from Instron Corporation, Canton, Massachusetts.
5. Test facility having a temperature of 23 ± 1°C, and a relative humidity of 50 ± 2 percent.

### Test Procedure

1. A sample to be tested is conditioned in the test facility for at least 4 hours prior to testing.
2. The load cell is calibrated and the software loaded.
3. The grips are installed on the tensile tester with the jaws closed.
4. The test condition for the tensile tester is set as follows:
   Crosshead speed = 500 millimeters/minute
   Full-scale load = 5 kilograms;
   Threshold = 5 percent;
   Fail criterion = 95 percent; and
   Gage length = 50 millimeters.
5. The weight of the clamp is tared out.
6. The ear portion on the back waist region of the article is inserted into the upper jaw such that the edge of the grip face is flush with the inner edge of the fastener material.
7. The front waist region of the article is inserted into the lower jaw such that the inner surface of the back waist region and the outer surface of the front waist region form a 180° angle. The lower jaw is closed.
8. The crosshead is started in motion.
9. The peak load of failure is recorded. It is intended that the mode of failure is that the back waist region of the diaper separates from the front waist region of the diaper.

### Shear Strength Test

This procedure is a tensile bench test to measure the shear force required to separate a mechanical fastening system that joins two materials. The shear force of separation is measured by determining load values as the two materials are pulled apart parallel to their plane of contact. The shear strength test values are an indication of how well the mechanical fastening system stays engaged against in-plane shear force. The sample is pulled in the tensile tester until the sample pulls apart. Shear strength is the peak load result. Shear strength is normalized by dividing by the contact area resulting in a force per area.

### Equipment

1. Tensile tester capable of obtaining a peak load and equipped with an appropriate load cell. A suitable tensile testing system is a Sintech Tensile Tester, commercially available from MTS Sintech, Research Triangle Park, North Carolina, under the trade designation Instron Model 4201 Tensile Tester with Sintech QAD (Quality Assurance Department) Software.
2. Software commercially obtained from MTS Sintech under the trade designation Sintech Testworks®.
3. Pnuematic-action grips commercially available from Instron Corporation, Canton, Massachusetts, under the trade designation "Instron Model 2712-004."
4. 1 by 4 inch grip faces, serrated, commercially available from Instron Corporation, Canton, Massachusetts.
5. Test facility having a temperature of 23 ± 1°C, and a relative humidity of 50 ± 2 percent.

### Test Procedure

1. A sample to be tested is conditioned in the test facility for at least 4 hours prior to testing.
2. The load cell is calibrated and the software loaded.
3. The grips are installed on the tensile tester with the jaws closed.
4. The test condition for the tensile tester is set as follows:
   Crosshead speed = 500 millimeters/minute;
   Full-scale load = 5 kilograms;
   Threshold = 5 percent;
   Fail criterion = 95 percent; and
   Gage length = 50 millimeters.
5. The weight of the clamp is tared out.
6. The back ear portion of the article is inserted Into the upper jaw such that the edge of the grip face is flush with the inner edge of the hook material.
7. The front waist region of the article is inserted into the lower jaw such that the inner surface of the back waist region and the inner surface of the front waist region are facing the same direction and are parallel to one another. The lower jaw is closed.
8. The crosshead is started in motion.
9. The peak load of failure is recorded, It is intended that the mode of failure is that the back waist region of the article separates from the front waist region of the article.

On the diaper 20, peel and shear strengths may be varied by (a) using different fastener materials along the lateral direction 40 of the fastener assembly 60 - 60b; (b) modifying the surface of the fastener assembly 60 - 60b along the lateral direction 40, by adding materials thereto, or by spraying, embossing, melting, deforming or the like; (c) modifying the inner ear surface 63 of the back ear portion, either by adding materials thereto, or by spraying, embossing, melting, deforming or the like; or (d) a combination of the above. Fastener assembly 60 - 60b and optional fastener materials added to inner ear surface 63 can include a variety of materials and surfaces known for mechanical engagement such as buttons, pins, snaps, adhesive tape fasteners, cohesives, mushroom-and-loop fasteners, and hook-and-loop fasteners, or the like. "Hooks" may be hook-like or post-like. Another possible fastener material includes reticulated foam, for example, FOAMEX Z6SCLY, manufactured by Foamex International Inc., Linwood, PA. The fastener assembly 60-60b is comprised of mechanical fastener materials that include components or members which protrude outwardly from the garment facing surface of diaper 20, e.g. an array of hooks or posts. Such protruding fastener materials may directly engage the inner ear surface 63.

Several examples of how connections having varying peel and shear strength can be realized are presented. However, it is not intended that the present invention be limited to these few examples. First, in the bow-shaped embodiment of Figs. 1-4, the middle portion 80 is a middle attachment zone that may comprise first material(s), and the outer regions 75 are side attachment zones that may comprise second material(s). In combination, the second material(s) in the side attachment zones have different peel or shear characteristics from the first material(s) in the middle attachment zones. It should be understood that the materials in the two side attachments zones may be different from each other in that they are not comprised of the same materials, or have the same peel and/or shear properties when attached to a common material. The inner surface 63 of the back ear portion 62 may be of the same material as the bodyside liner 44.

Rather than having more than one material in each attachment zone, there may be only a single first material and a single second material. The first and second materials may be integrally connected (e.g. cut from a single sheet) or pieced together and either attached together, abutted or positioned in close proximity. Desirably, the first and second materials may be a hook material that has been modified in either the middle portion or the outer regions to make the peel and or shear values differ when the back ear portions 62 are attached thereto. For example, an adhesive material may be added to the middle portion 80 to increase the peel strength.

The second material(s), in combination, have different peel or shear characteristics from the first material(s). In combination, the second material(s) in the side attachment zones have different peel or shear characteristics from the first material(s) in the middle attachment zones. It should be understood that the materials in the two side attachments zones may be different from each other in that they are not comprised of the same materials, or they may have the same peel and/or shear properties when attached to a common material. Of course, rather than having more than one material in each attachment zone, there may be only a single first material and a single second material. The inner surface 63 of the back ear portion 62 may be made from the same material as the bodyside liner 44.

In the third example as seen in Figs. 5 and 6, the middle portion 185 may comprised of first material(s), and fastener components 184 may be comprised of second material(s). In combination, the second material(s) in the side attachment zones have different peel or shear characteristics from the first material(s) in the middle attachment zones. It should be understood that the materials in the two side attachments zones may be different from each other In that they are not comprised of the same materials, or they may have the same peel and/or shear properties when attached to a common material. Of course, rather than having more than one material in each attachment zone, there may be only a single first material and a single second material. Further, each separate element 184 may be comprised of a different material. Again, the inner surface 63 of the back ear portion 62 may be of the same material as the bodyside liner 44.

In an alternative embodiment (not shown), the diaper 20 may have the back ear portion 62 located where the current front ear portion 64 is located, and the front ear portion 64 located where the back ear portion 62 is located, and the mechanical fastener assembly 60-60b may be disposed on the back surface waist region 24 instead of the front waist region 22 of the diaper 20. In addition, separate tab members may extend form the back ear portion 62 (not shown).

In operation, the diaper 20 of the present invention may be pro-fastened so that it may be applied and removed as either a diaper or a pant. If the diaper 20 is applied In diaper fashion, the wearer or caretaker aligns the pant about the body and fastens the diaper 20 by overlapping and then attaching the inner surface 63 of back ear portions 62 to the fastener assembly 60. If there is no addition or modification or the inner surface 63, there is no need for the wearer/caretaker to align any particular area if the inner surface 63 with the fastener assembly 60. If there is an addition or modification to the inner surface 63, it is desirable that the area of the addition or modification be sized such that the wearer/caretaker does not need to take additional effort to align these areas with portions of the fastener assembly 60. Once the side seam 76 is formed by attaching the proximal region 96 of the back ear portion to the fastener assembly, distal regions 98 may be repositioned to adjust the fit and/or appearance of the diaper 20.

One of skill in the art will readily appreciate that the various fastener assemblies and ears described herein may be combined to arrive at a number of configurations not illustrated herein, yet quite suitable for use in fastening diapers and or pants. Having described the invention in rather full detail, it will be readily apparent that various changes and modifications can be made without departing from the invention. All of such changes and modifications are contemplated as being within the scope of the invention as defined by the appended claims and any equivalents thereto.

## Claims

1. A disposable garment (20) having a front waist region (22), a rear waist region (24) and a crotch region (26) which extends between and connects said front waist region (22) and said rear waist region (24), the disposable garment (20) comprising:
a body-facing surface (34);
a garment-facing surface (36); and
a fastener assembly (60) comprising:
two side attachment zones (75) transversely disposed in juxtaposed relation on the garment-facing surface (36) of the front waist region (22); and
a middle attachment zone (80) disposed on the garment-facing surface of the front waist region (22) between the two side attachment zones (75), wherein said middle attachment zone (80) contains at least one first fastener material and said side attachment zones (75) contain at least one second fastener material,
**characterized in that**:
said at least one first fastener material and said at least one second fastener material are comprised of a plurality of protruding members for engaging said body-facing surface;
in use, said side attachment zones (75) engage the body-facing surface (34) at a significantly different normalized peel strength than said middle attachment zone (80); and
the peel strength between the middle attachment zone (80) and the body-facing surface (34) is less than the peel strength between the side attachment zones (75) and the body-facing surface (34).

2. The disposable garment (20) of claim 1, wherein at least a portion of the body-facing surface (34) is modified to change the peel strength when it is attached to the fastener assembly (60).

3. The disposable garment (20) of claim 1 or 2, wherein the at least one first fastener material is comprised of mushroom fasteners, reticulated foam fasteners, or hook fasteners.

4. The disposable garment (20) of any of claims 1 to 3, wherein the at least one second fastener material is comprised of mushroom fasteners, reticulated foam fasteners, or hook fasteners

5. The disposable garment (20) of any preceding claim, wherein the at least one first fastener material and the at least one second fastener material are equivalent.

6. The disposable garment (20) of any preceding claim, wherein the side attachment zones (75) each have a first longitudinal length that decreases in a lateral direction (40) toward a longitudinal axis of the garment (20), and a second longitudinal length measured at the longitudinal axis of the garment (20), wherein an average of the first longitudinal length is greater that the second longitudinal length.

7. The disposable garment (20) of any preceding claim, further comprising:
an outer cover (42);
a bodyside liner (44); and
an absorbent core (28) disposed between the bodyside liner (44)and the outer cover (42).

8. The disposable garment (20) of claim 1, further comprising a pair of back ear portions (62) extending from the rear waist region (24), and configured so that, in use, the pair of back ear portions (62) each engage at least a portion of one of the side attachment zones (75) and at least a portion of the middle attachment zone (80).

9. The disposable garment (20) of claim 8, wherein the at least one first fastener material is comprised of mushroom fasteners, reticulated foam fasteners, or hook fasteners.

10. The disposable garment (20) of claim 8 or 9, wherein the at least one second fastener material is comprised of mushroom fasteners, reticulated foam fasteners, or hook fasteners.

11. The disposable garment (20) of any of claims 8 to 10, wherein the at least one first fastener material is mechanically treated to reduce the shear strength of the fastener material.

12. The disposable garment (20) of claim 11, wherein the protruding members of the first fastener material or the second fastener material are treated with an adhesive.

13. The disposable garment (20) of claim 12, wherein the protruding members of the first fastener material or the second fastener material are hooks.

14. The disposable garment (20) of any of claims 8 to 10, wherein the pair of back ear portions (62) have modified body-facing surfaces and the at least one first fastener material and the at least one second fastener material have equivalent surface characteristics.

15. The disposable garment (20) of any of claims 8 to 10, wherein the pair of back ear portions (62) have uniform body-facing surfaces, and the at least one first fastener material is different than the at least one second fastener material.

16. The disposable garment (20) of any of claims 8 to 15, further comprising: an absorbent core (28) disposed between the body-facing material and the outer cover (42).

17. The disposable garment (20) of any of claims 8 to 16, wherein the body-facing material and a body-facing surface of the pair of back ear portions (62) are comprised of a liner material.

## Patentansprüche

1. Einweg-Bekleidungsstück (20), welches einen vorderen Taillenbereich (22) einen hinteren Taillenbereich (24) und einen Schrittbereich (26) aufweist, welcher sich zwischen dem vorderen Taillenbereich (22) und dem hinteren Taillenbereich (24) erstreckt und diese verbindet, wobei das Einweg-Bekleidungsstück (20) umfasst:
eine körperzugewandte Oberfläche (34);
eine kleidungszugewandte Oberfläche (36); und
eine Verschlussanordnung (60), welche umfasst:
zwei seitliche Befestigungszonen (75), welche quer in einer nebeneinander angeordneten Beziehung auf der kleidungszugewandten Oberfläche (36) des vorderen Taillenbereichs (22) angeordnet sind; und
eine mittlere Befestigungszone (80), die auf der kleidungszugewandten Oberfläche des vorderen Taillenbereichs (22) zwischen den zwei seitlichen Befestigungszonen (75) angeordnet ist, wobei die mittlere Befestigungszone (80) mindestens ein erstes Verschlussmaterial und die seitlichen Befestigungszonen (75) mindestens ein zweites Verschlussmaterial enthalten,
**dadurch gekennzeichnet, dass**:
das mindestens eine erste Verschlussmaterial und das mindestens eine zweite Verschlussmaterial eine Vielzahl von hervorstehenden Elementen umfassen zum Verbinden mit der körperzugewandten Oberfläche;
in Verwendung sich die seitlichen Befestigungszonen (75) mit der körperzugewandten Oberfläche (34) mit einer signifikant verschiedenen normalisierten Abziehfestigkeit verbinden als die mittlere Befestigungszone (80); und
die Abziehfestigkeit zwischen der mittleren Befestigungszone (80) und der körperzugewandten Oberfläche (34) geringer ist als die Abziehfestigkeit zwischen den seitlichen Befestigungszonen (75) und der körperzugewandten Oberfläche (34).

2. Einweg-Bekleidungsstück (20) gemäß Anspruch 1, wobei mindestens ein Teil der körperzugewandten Oberfläche (34) modifiziert ist, um die Abziehfestigkeit zu ändern, wenn sie an der Verschlussanordnung (60) befestigt ist.

3. Einweg-Bekleidungsstück (20) gemäß Anspruch 1 oder 2, wobei das mindestens eine erste Verschlussmaterial Mushroom-Typ-Verschlüsse, Verschlüsse aus retikuliertem Schaum oder Hakenverschlüsse umfasst.

4. Einweg-Bekleidungsstück (20) gemäß einem der Ansprüche 1 bis 3, wobei das mindestens eine zweite Verschlussmaterial Mushroom-Typ-Verschlüsse, Verschlüsse aus retikuliertem Schaum oder Hakenverschlüsse umfasst.

5. Einweg-Bekleidungsstück (20) gemäß einem der vorhergehenden Ansprüche, wobei das mindestens eine erste Verschlussmaterial und das mindestens eine zweite Verschlussmaterial äquivalent sind.

6. Einweg-Bekleidungsstück (20) gemäß einem der vorherigen Ansprüche, wobei jede der seitlichen Befestigungszonen (75) eine erste Längslänge aufweist, welche sich in einer seitlichen Richtung (40) in Richtung einer Längsachse des Bekleidungsstücks (20) verringert, und eine zweite Längslänge aufweist, welche an der Längsachse des Bekleidungsstücks (20) gemessen wird, wobei ein Durchschnitt der ersten Längslänge größer ist als die zweite Längslänge.

7. Einweg-Bekleidungsstück (20) gemäß einem der vorherigen Ansprüche, welches des Weiteren umfasst:
eine äußere Deckschicht (42);
eine körperseitige Auskleidung (44); und
einen absorptionsfähigen Kern (28), der zwischen der körperseitigen Auskleidung (44) und der äußeren Deckschicht (42) angeordnet ist.

8. Einweg-Bekleidungsstück (20) gemäß Anspruch 1, welches des Weiteren ein Paar von hinteren Ohrteilen (62) umfasst, die sich von dem hinteren Taillenbereich (24) erstrecken und eingerichtet sind, so dass sich, bei Verwendung, jedes des Paars von Ohrteilen (62) mit mindestens einem Teil einer der seitlichen Befestigungszonen (75) und mit mindestens einem Teil der mittleren Befestigungszone (80) verbindet.

9. Einweg-Bekleidungsstück (20) gemäß Anspruch 8, wobei das mindestens eine erste Verschlussmaterial Mushroom-Typ-Verschlüsse, Verschlüsse aus retikuliertem Schaum oder Hakenverschlüsse umfasst.

10. Einweg-Bekleidungsstück (20) gemäß Anspruch 8 oder 9, wobei das mindestens eine zweite Verschlussmaterial Mushroom-Typ-Verschlüsse, Verschlüsse aus retikuliertem Schaum oder Hakenverschlüsse umfasst.

11. Einweg-Bekleidungsstück (20) gemäß einem der Ansprüche 8 bis 10, wobei das mindestens eine erste Verschlussmaterial mechanisch behandelt ist, um die Scherfestigkeit des Verschlussmaterials zu reduzieren.

12. Einweg-Bekleidungsstück (20) gemäß Anspruch 11, wobei die hervorstehenden Elemente des ersten Verschlussmaterials oder des zweiten Verschlussmaterials mit einem Haftmittel behandelt sind.

13. Einweg-Bekleidungsstück (20) gemäß Anspruch 12, wobei die hervorstehenden Elemente des ersten Verschlussmaterials oder des zweiten Verschlussmaterials Haken sind.

14. Einweg-Bekleidungsstück (20) gemäß einem der Ansprüche 8 bis 10, wobei das Paar von hinteren Ohrteilen (62) modifizierte körperzugewandte Oberflächen aufweist und das mindestens eine erste Verschlussmaterial und das mindestens eine zweite Verschlussmaterial äquivalente Oberflächencharakteristiken aufweisen.

15. Einweg-Bekleidungsstück (20) gemäß einem der Ansprüche 8 bis 10, wobei das Paar von hinteren Ohrteilen (62) einheitliche körperzugewandte Oberflächen aufweist, und das mindestens eine erste Verschlussmaterial verschieden ist von dem mindestens einen zweiten Verschlussmaterial.

16. Einweg-Bekleidungsstück (20) gemäß einem der Ansprüche 8 bis 15, welches des Weiteren umfasst: einen absorptionsfähigen Kern (28), der zwischen dem körperzugewandten Material und der äußeren Deckschicht (42) angeordnet ist.

17. Einweg-Bekleidungsstück (20) gemäß einem der Ansprüche 8 bis 16, wobei das körperzugewandte Material und eine körperzugewandte Oberfläche des Paars von hinteren Ohrteilen (62) ein Auskleidungsmaterial umfassen.

## Revendications

1. Vêtement jetable (20) ayant un région taille avant (22), une région taille arrière (24) et une région entrejambes (26) qui s'étend entre ladite région taille avant (22) et ladite région taille arrière (24) et les relie, ce vêtement jetable (20) comprenant :
une surface faisant face au corps (34) ;
une surface faisant face au vêtement (36) ; et
un ensemble de fixation (60) comprenant :
deux zones de fixation latérales (75) disposées transversalement dans un rapport juxtaposé sur la surface faisant face au vêtement (36) de la région taille avant (22) ; et
une zone de fixation médiane (80) disposée sur la surface faisant face au vêtement de la région taille avant (22) entre les deux zones de fixation latérales (75), ladite zone de fixation médiane (80) contenant au moins une première matière de fixation et lesdites zones de fixation latérales (75) contenant au moins une deuxième matière de fixation,
**caractérisé en ce que** :
ladite au moins une première matière de fixation et ladite au moins une deuxième matière de fixation sont constituées d'une pluralité d'éléments faisant saillie destinés à s'engager avec ladite surface faisant face au corps ;
en cours d'utilisation, lesdites zones de fixation latérales (75) s'engagent avec la surface faisant face au corps (34) avec une force de détachement normalisée sensiblement différente de celle de ladite zone de fixation médiane (80) ; et
la force de détachement entre la zone de fixation médiane (80) et la surface faisant face au corps (34) est inférieure à la force de détachement entre les zones de fixation latérales (75) et la surface faisant face au corps (34).

2. Vêtement jetable (20) selon la revendication 1, dans lequel au moins une partie de la surface faisant face au corps (34) est modifiée de façon à changer la force de détachement lorsqu'elle est attachée à l'ensemble de fixation (60).

3. Vêtement jetable (20) selon la revendication 1 ou 2, dans lequel l'au moins une première matière de fixation est constituée par des fixations velcro, des fixations en mousse réticulée, ou des fixations à crochet.

4. Vêtement jetable (20) selon l'une quelconque des revendications 1 à 3, dans lequel l'au moins une deuxième matière de fixation est constituée par des fixations velcro, des fixations en mousse réticulée ou des fixations à crochet.

5. Vêtement jetable (20) selon l'une quelconque des revendications précédentes, dans lequel l'au moins une première matière de fixation et l'au moins une deuxième matière de fixation sont équivalentes.

6. Vêtement jetable (20) selon l'une quelconque des revendications précédentes, dans lequel les zones de fixation latérales (75) ont chacune une première longueur longitudinale qui diminue dans une direction latérale (40) vers un axe longitudinal du vêtement (20), et une deuxième longueur longitudinale mesurée à l'axe longitudinal du vêtement (20), dans lequel une moyenne de la première longueur longitudinale est plus grande que la deuxième longueur longitudinale.

7. Vêtement jetable (20) selon l'une quelconque des revendications précédentes, comprenant :
un revêtement extérieur (42) ;
une doublure côté corps (44) ; et
un centre absorbant (28) disposé entre la doublure côté corps (44) et le revêtement extérieur (42).

8. Vêtement jetable (20) selon la revendication 1, comprenant en outre une paire de parties oreilles arrière (62) s'étendant depuis la région taille arrière (24), et configurées de manière à ce que, en cours d'utilisation, la paire de parties oreilles arrière (62) s'engagent chacune avec au moins une partie d'une des zones de fixation latérales (75) et avec au moins une partie de la zone de fixation médiane (80).

9. Vêtement jetable (20) selon la revendication 8, dans lequel l'au moins une première matière de fixation est constituée par des fixations velcro, des fixations en mousse réticulée ou des fixations à crochet.

10. Vêtement jetable (20) selon la revendication 8 ou 9, dans lequel l'au moins une deuxième matière de fixation est constituée par des fixations velcro, des fixations en mousse réticulée, ou des fixations à crochet.

11. Vêtement jetable (20) selon l'une quelconque 8 à 10, dans lequel l'au moins une première matière de fixation est traitée mécaniquement pour réduire la résistance au cisaillement de la matière de fixation.

12. Vêtement jetable (20) selon la revendication 11, dans lequel les éléments faisant saillie de la première matière de fixation ou de la deuxième matière de fixation sont traités avec un adhésif.

13. Vêtement jetable (20) selon la revendication 12, dans lequel les éléments faisant saillie de la première matière de fixation ou de la deuxième matière de fixation sont des crochets.

14. Vêtement jetable (20) selon l'une quelconque des revendications 8 à 10, dans lequel la paire de parties oreilles arrière (62) ont des surfaces faisant face au corps modifiées et l'au moins une première matière de fixation et l'au moins une deuxième matière de fixation ont des caractéristiques de surface équivalentes.

15. Vêtement jetable (20) selon l'une quelconque des revendications 8 à 10, dans lequel la paire de parties oreilles arrière (62) ont des surfaces faisant face au corps uniformes et l'au moins une première matière de fixation est différente de l'au moins une deuxième matière de fixation.

16. Vêtement jetable (20) selon l'une quelconque des revendications 8 à 15, comprenant en outre : un centre absorbant (28) disposé entre la matière faisant face au corps et le revêtement extérieur (42).

17. Vêtement jetable (20) selon l'une quelconque des revendications 8 à 16, dans lequel la matière faisant face au corps et une surface faisant face au corps des parties oreilles arrière (62) sont constituées par un matière de doublure.
